# EUROPEAN PATENT SPECIFICATION

(11) **EP 3 265 000 B1**
(45) Date of publication and mention of the grant of the patent: **27.03.2019**
(21) Application number: 16713052.5
(22) Date of filing: 26.02.2016
(51) Int. Cl.: A61B 17/16

(54) **A SURGICAL INSTRUMENT FOR SCRAPING AND COLLECTING PARTICLES OF BONE**
CHIRURGISCHES INSTRUMENT ZUR AUSSCHABUNG UND ENTNAHME VON KNOCHENPARTIKELN
INSTRUMENT CHIRURGICAL POUR RACLER ET COLLECTER DES PARTICULES D'OS

(30) Priority: 03.03.2015 IT RE20150014
(43) Date of publication of application: 10.01.2018
(73) Proprietor: C.G.M. S.P.A., 42015 Correggio (RE) (IT)
(72) Inventor: PARMIGIANI, Corrado, Saverio, 42015 Correggio (IT)
(74) Representative: Corradini, Corrado
(86) International application number: PCT/IB2016/000199
(87) International publication number: WO 2016/139522

(56) References cited:
- EP-A1- 1 829 486
- EP-A2- 1 749 487

## Description

### TECHNICAL FIELD

The present invention relates to techniques of reconstruction and regenerative removal of bone tissues in oral and maxillo-facial orthopaedic surgery, plastic surgery, periodontal surgery and implant surgery as well as techniques of plastic surgery on bones.

### PRIOR ART

Instruments have been developed in the past for collecting bone material in various zones of the skeletal structure with the aim of obtaining particles of dimensions suitable for regenerative tissue biological requirements.

These instruments have enables increasing autologous bone removal techniques and the treatment of the collected bone material, succeeding in obtaining bone particles for filling bone defects or for increasing skeletal structures.

In recent decades, the collecting methods have been refined thanks to the introduction on the market of instruments for removing and collecting particles (flakes or shavings) by scraping from bones, which instruments comprise a handle bearing a scraper blade located at the distal end of the instrument (front) and a chamber for collecting the removed material.

These devices enable removal of surface cortical bone with a special scraping blade with which the instrument is equipped, which generates particles, in the form of thin flakes or shavings, of bone, which are directly collected internally of the collecting chamber.

The high technological level of the scraper blade enables obtaining, with a slight pressure, an optimal and controllable cut. The collecting of the scraped bone particles is done via a passage opening (slit) located at the base of the blade, which conveys the particles internally of the collecting chamber defined by a protected compartment, for temporary storing. During the collecting step, the bone particles are mixed with blood to form a high-density bone concentrate, ideal as a filler in regenerative techniques.

An instrument, illustrated in Italian patent application no.IT2005RE000098 in the name of the same Applicant, discloses a surgical instrument for scraping and collecting bone particles comprising a handle bearing a scraper blade located at the distal end, having a tubular shape with a closed circular section, which delimits a collecting chamber of the removed material, enabling passage of the scraped particles inside the chamber, in which the instrument comprises a collecting means of the particles having a tubular syringe shape in which a piston slides, insertable and extractable from the collecting chamber for collecting the particles from the chamber.

An important advantage deriving from this type of instrument is that it makes collecting the particles from the collecting chamber simple and effective, together with the transfer thereof to the receiving site rapidly and aseptically, and avoiding the need for any further manipulations. In fact, the collecting means is used as a sort of syringe for directly introducing the material collected into the receiving site; this is particularly advantageous in a case in which it is necessary to pour the particles into narrow sites, or through a hole made in a bone tissue of the same donor (typically for raising a maxillary sinus); thanks to the shape of the collecting means, the material contained therein can effectively and easily be poured into the cavity of the hole, even to a considerable depth.

Document EP1749487 A2 discloses an exemplary surgical instrument for scraping and collecting particles of bone.

### DISCLOSURE OF THE INVENTION

An aim of the invention is to perfect this type of instrument so as to enable use of a collecting means which functions like a syringe, having a smaller diameter; this is with the aim of enabling introduction of the particles into sites that otherwise would be difficult to reach.

A further aim of the invention is to obviate a drawback connected to the fact that the tubular structure of the handle causes annoying noises due to vibrations during the bone-scraping operations.

These and other aims are attained by the invention as it is characterised in the claims.

The construction materials of the device are not critical, with the exception of the blade which obviously must be made of steel. Both the handle and the mobile wall are preferably made of synthetic resin suitable for medical uses, such as for example polycarbonate or acrylonitrile/butadiene/styrene.

### BRIEF DESCRIPTION OF THE DRAWINGS

The invention is described in detail in the following with the aid of the accompanying figures of the drawings which illustrate an embodiment thereof purely by way of non-limiting example.
Figure 1 is a perspective view, in three-quarter view, of the instrument of the invention in a closed configuration.
Figure 1A is a front view of figure 1.
Figure 2 is a side view of figure 1.
Figure 3 is a section according to a vertical axial plane of figure 2.
Figure 3A is the same view as in figure 1, with the instrument in a start configuration of opening.
Figure 3B is a section along plane B-B of figure 3.
Figure 4 is a detail IV of figure 3 in larger-scale detail.
Figure 5 is the same perspective view as in figure 1, with the instrument in an open configuration.
Figure 6 is a section according to a vertical axial plane of figure 5 of the tool in the open configuration.

### PREFERRED EMBODIMENT OF THE INVENTION

The surgical instrument for scraping and collecting particles of bone, comprises an elongate handle (10), having a distal end bearing a blade (20).

In a preferred embodiment the handle has an elongate shape, substantially straight and an upper external face, a section of which is convex, and a lower internal face, a section of which is concave.

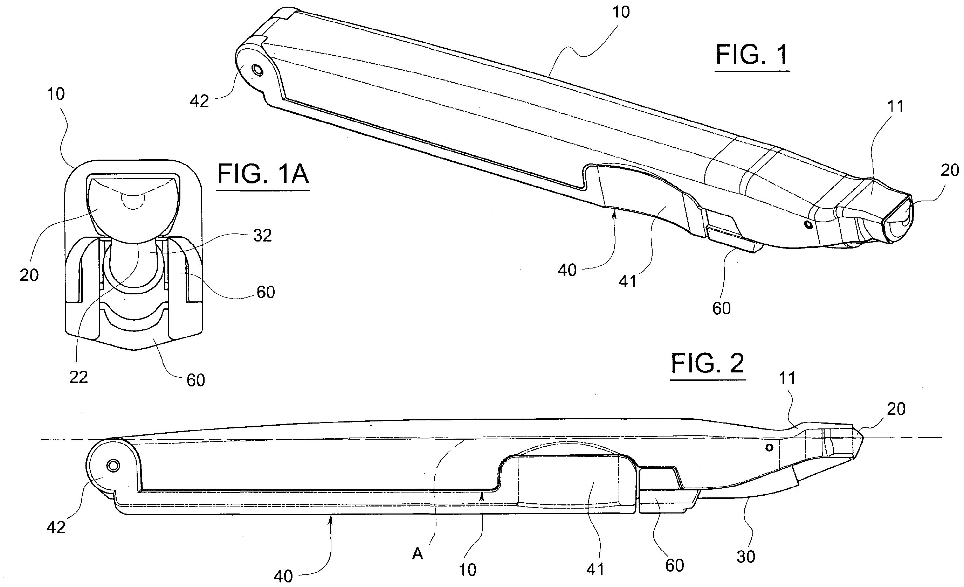

The terms "upper" and "lower" used in the present description relate to the position of the instrument in the closed configuration, illustrated in figures 1-3. The blade 20 has a scraper face 21 and an external scraping edge 22, projecting transversally and inferiorly with respect to the distal end 11 of the handle.

In a preferred embodiment, illustrated in the figures of the drawings, the blade 20 is joined in a single body with a shank 25 solidly incorporated internally of the distal end 11 of the handle, with the axis parallel and substantially coaxial with the longitudinal axis A of the end 11, solidly constrained to the material thereof.

The blade 20 has a substantially flat scraping face 21, facing rearwards, i.e. facing the distal end 11, which is further facing downwards (in the conventional geometric arrangement illustrated in figures 1-3) the lower edge of which projects transversally downwards, beyond the distal end 11, and defines the scraping edge 22. In particular, the scraping face 21 is perpendicular to the axis of the shank 25.

The instrument comprises a collecting compartment 30 of the scraped material which has a tubular shape and comprises a collecting chamber 31 having a constant transversal section and a distal end 32 which has a mouth 34 communicating with outside.

In the invention, the collecting compartment 30 is constrained to the handle 10, and is able to assume two different operating configurations:
- a first operating configuration in which the compartment 30 directly rests against an internal face of the handle 10, with the distal end 32 thereof located adhering to the distal end 11 of the handle and facing the scraping face, in proximity of the scraping edge 22, so as to receive the scraped material in inlet, and
- a second operating configuration in which the compartment 30 is located at a distance from the distal end 11 of the handle and a mouth thereof is located distant from the blade 20 and from the handle 10.

In a preferred embodiment, illustrated in the figures of the drawings, the collecting compartment 30 is constrained at a proximal end 33 thereof to the proximal portion 12 of the handle 10, by means of a constraint which enables reciprocal rotation of the handle 10 and the compartment 30.

In greater detail, the collecting compartment 30 is solidly joined to a rear support stem 40, a rear end 42 of which is hinged to the proximal end 12 of the handle 10, by means of a hinge 44 having a horizontal rotation axis that is transversal with respect to the horizontal axis A of the handle 10.

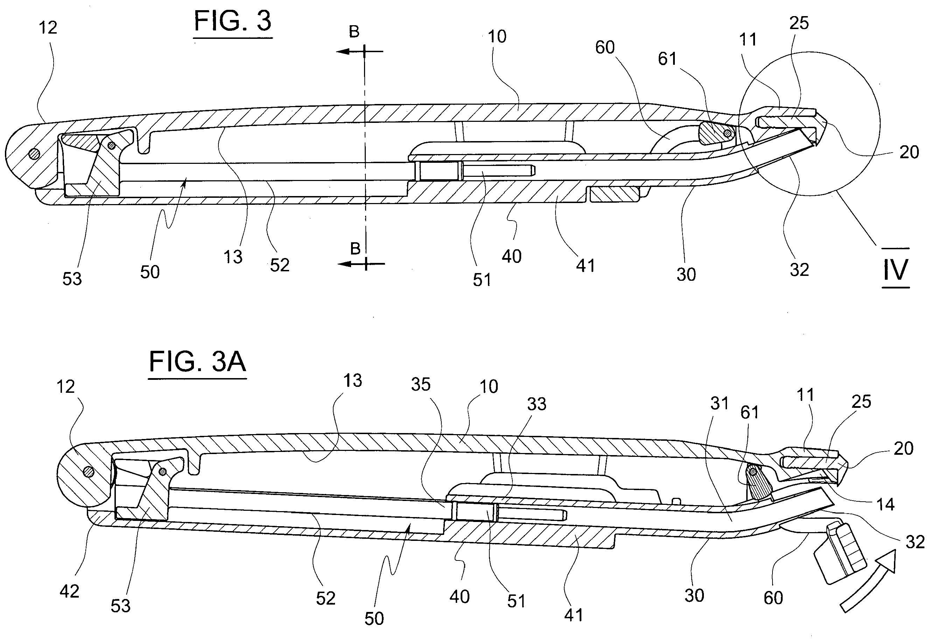

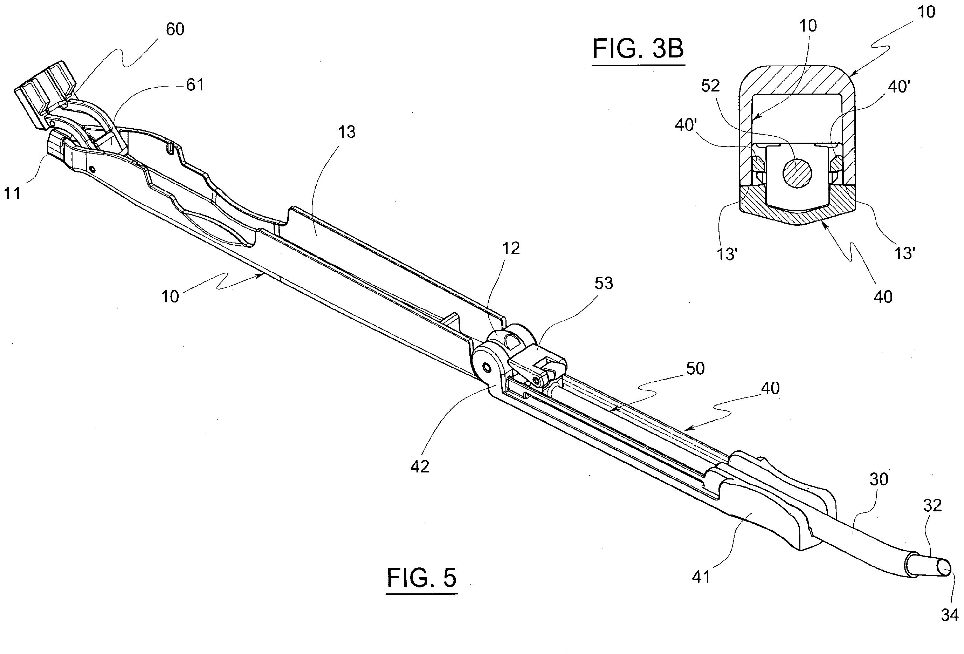

The rear stem 40 has a shorter length with respect to the handle 10 and is conformed such as to be arranged restingly on the handle 10, and in part such as to co-penetrate the internal face of the handle 10, when the compartment 30 is in the first operating configuration (figures 1-3). The handle has a transversal section having substantially over all a length extension thereof a concave shape (U-shape), with the concavity thereof facing downwards; the internal surface defined by the concavity constitutes the internal face 13 of the handle (see in particular figure 3B).

In detail (see figure 3B), the support stem 40 comes to rest against (when the compartment 30 is in the first operating configuration) against the lower ends 13' of the U-section of the handle, and simultaneously penetrates for a short portion 40' thereof defines by two protuberances 40' to enter and joint in the delimited cavity of the face 13 of the handle. Owing to these characteristics, the rear stem 40 also functions as a structural stiffening element of the handle 10.

The compartment 30 is solidly joined to the distal portion 41 of the stem 40 and prolongs therefrom, projecting forward up to bringing the distal end 32 thereof to adhere to the distal end of the instrument and facing the scraping face in proximity of the scraping edge 22.

The collecting compartment is provided with a piston 50 having a rod 51 which is able to slide along the whole longitudinal extension of the collecting chamber 31 of the compartment and an activating plunger 52 which projects from the chamber 31 through a proximal mouth 35 of the compartment, located at the rear end thereof.

The rear end 53 (proximal) of the stem is shaped so as to be easily pushed by an operator's finger and to slide along the internal face of the rear stem 40.

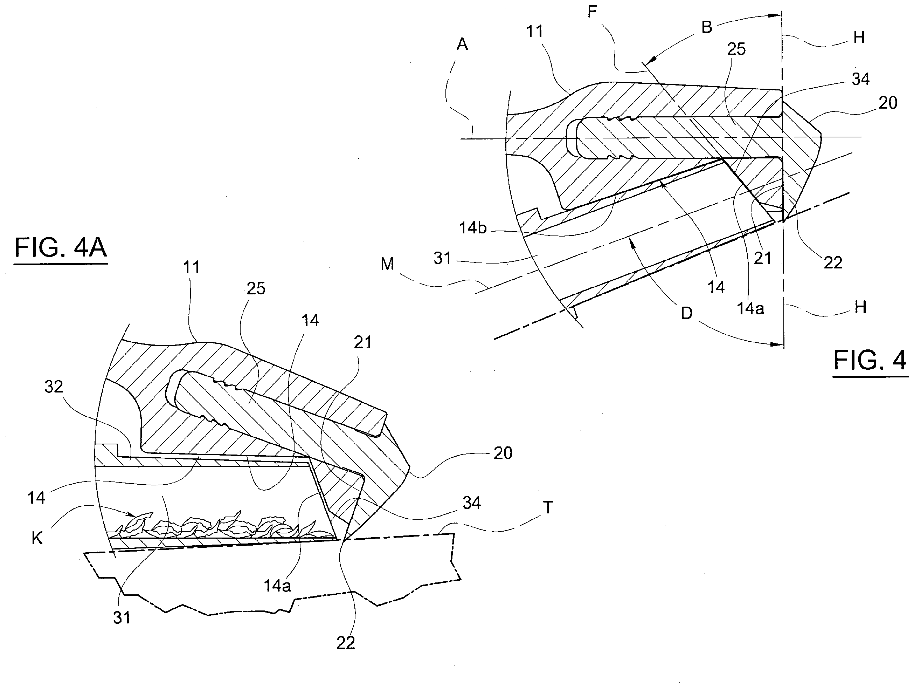

The distal end 11 of the handle exhibits a smaller concavity 14 facing inferiorly, conformed complementarily with the upper portion of the distal end 32 of the compartment and with the mouth 34 thereof, so that the surface of the upper portion of the compartment can adhere to the smaller concavity 14, when the compartment 30 is in the first operating configuration (see figure 4). In detail, the smaller concavity 14 is delimited by a rear face 14b with an arched section, which adheres to the surface of the upper portion of the distal end 32, and by a flat front face 14a, which adheres to the upper portion of the communicating mouth (34) of the compartment, so as to close the majority of the mouth (see figure 4). In particular, the plane defined by the communicating mouth 34 of the compartment coincides with the plane defined by the front face 14a of the smaller concavity.

The plane F defined by the communication 34 of the compartment forms an angle B with the plane H defined by the scraping face 21 of the blade 20, a value of which is around 40 degrees, and preferably exactly 40 degrees. The axis M of the distal end 32 of the compartment forms an acute angle of about 20 degrees with the axis A of the handle, i.e. it forms an angle D with the plane H defined by the scraping face 21 of the blade 20 a value of which is around 70 degrees, and preferably exactly 70 degrees.

The instrument comprises a lever 60 hinged to the handle 10, which surrounds the compartment 30 when the compartment is in the first operating configuration.

The lever 60 has two end positions, in which:
- in the first end position (illustrated in figure 3), the lever rests against the compartment 30, in turn positioned restingly against the internal face 13 of the handle 10, in the first operating configuration,
- in the second end position (illustrated in figure 3A), the lever is rotated with respect to the first position and raised by the handle 10, and in turn raises the compartment 30, distancing it (with a small rotation) from the handle 10 where it rested during the first end position of the lever.

In use, to scrape and collect bone particles, the instrument is arranged in the first operating configuration (figures 1-3), gripping it with a hand by the handle 10, so that the distal portion of the instrument and in particular the distal end 11 of the handle, projects outwards from the fingers of the hand. During the scraping, the distal end 32 of the compartment is located parallel to and resting on the surface of the bone T (as illustrated in figure 4A).

When the scraping edge 22 is pressed downwards against the surface of the bone T the distal part of the handle 10 flexes elastically below the pushing of the operator's hand and the reaction produced by the resting of the blade 20 on the bone T, forming a slight arch the concavity of which faces upwards.

The distal end 32 of the compartment, which is not directly constrained to the operator's fingers, nor to the blade 20, rests on the surface of the bone and detaches (for a few tenths or hundreds of millimetres) from the front face 14a of the smaller concavity 14, against which it was previously resting, detaching the mouth 34 from the smaller concavity 14.

During this step, by pulling the instrument backwards while the scraping edge 22 is pressed against the bone, the material scraped from the scraping edge 22 (schematised in figure 4A by shavings K) is pushed towards the scraper face 21, internally of the collecting compartment 31, via the mouth 34 which in this step is open. The scraped shavings pushed from the scraping edge 22 are pushed internally of the chamber 31.

In experimental use, it has been found that the shapes and characteristics of the distal portion of the instrument described in the foregoing lead to an extremely effective operating functionality during the step of scraping and collecting bone particles.

When the action produced by the hand on the instrument ceases, which pushes the blade 20 against the bone, the mechanical stresses on the distal portion of the handle 10 also cease and the handle returns into its natural configuration and the mouth 34 of the compartment newly goes to rest against the faces 14a and 14b, almost entirely isolating the chamber 31 from the external environment. This contact preferably happens with a certain preload realised during the construction of the instrument, so as better to close the mouth 34.

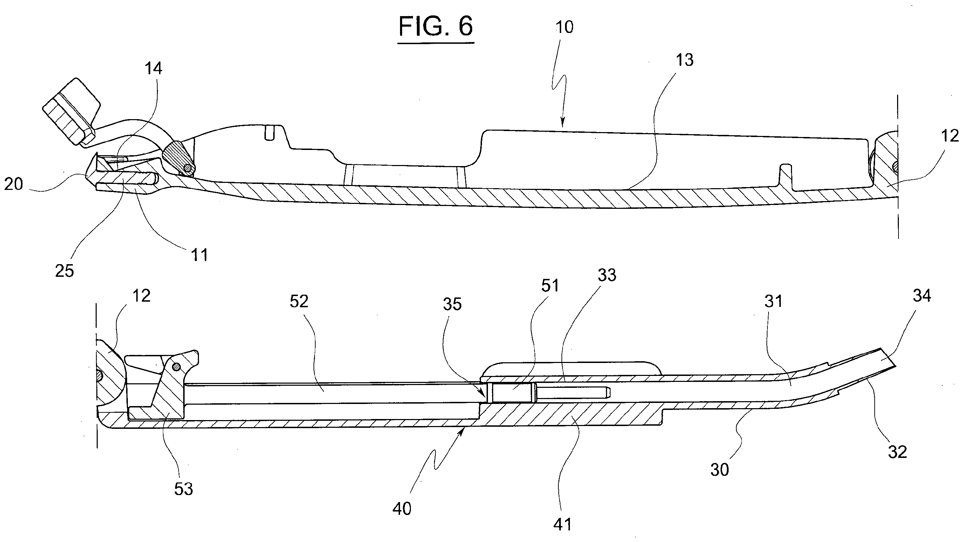

After the bone particles have been collected in the distal part of the chamber 31, the compartment 30 can be distanced from the handle 10 (bringing it into the second operating configuration) (figures 5 and 6), by arranging the handle 10, with a rotation about the axis of the hinge 44, aligned or nearly aligned with the rear stem 40 and with the compartment 30, on the opposite side with respect to the hinge 44.

The rear stem 40 and the compartment 30 can be used as a syringe, which contains the particles of bone in the chamber 31 thereof, operatively independent of the handle 10, and thus extremely easy to pour, by acting on the piston 50, the bone particles into narrow sites or through a hole presented in a bone tissue of the donor (typically to construct a raising of the maxillary sinus).

The lever 60 is held in the first operating position while the instrument is in the first operating configuration so as to scrape and collect shavings of bone; during this step the lever 60 is useful for stabilising the position of the compartment 30 against the internal face of the handle 10.

The lever 60 is further very useful for detaching the compartment 30 from the handle 10 when the compartment 30 is in the first operating configuration: by raising the lever 60 into the second operating position (figure 3A), the compartment 30 is raised with respect to the handle 10, thanks to the action of the body 61 of the lever, and can more easily be gripped by the operator's fingers.

Obviously numerous modifications of a practical-applicational nature can be made to the present invention without forsaking the scope of the inventive idea as claimed in the following.

## Claims

1. A surgical instrument for scraping and collecting particles of bone, comprising an elongate handle (10), having a distal end bearing a blade (20) having a scraper face (21), facing rearwards, and an external scraping edge (22), projecting transversally externally with respect to the distal end,
and a collecting compartment (30) of the scraped material, wherein the collecting compartment (30) has a tubular shape and comprises a collecting chamber (31) having a constant transversal section and a distal end (32) which has a mouth (34) communicating with outside,
the collecting compartment (30) is connected with a proximal end (33) thereof to a proximal portion (12) of the handle (10), by means of a constraint which enables reciprocal rotation of the handle (10) and the compartment (30), and is able to assume two different operating configurations:
- a first operating configuration in which the collecting compartment (30) directly rests against an internal face (13) of the handle (10) facing the collecting compartment (30), with the distal end (32) thereof located adhering to the distal end (11) and facing the scraping face (21) of the blade, in proximity of the scraping edge (22), so as to receive the scraped material in inlet, and
- a second operating configuration in which the compartment (30) is located at a distance from the distal end (11) of the handle and a mouth (34) thereof is located distant from the blade (20) and from the handle (10), and wherein the surgical instrument comprises a piston (50) associated to the collecting compartment (30), wherein the piston (50) has a rod (51) which slides along the collecting chamber (31) of the collecting compartment (30) and an activating plunger (52) which projects from the collecting chamber (31) at the proximal end of the collecting compartment (30).

2. The instrument of claim 1, wherein the distal end (11) of the handle (10) exhibits a smaller concavity (14) facing toward the collecting compartment (30), conformed complementarily with the upper portion of the distal end (32) of the compartment and with the mouth (34) thereof, so that the external surface of the portion can adhere to the smaller concavity (14), when the compartment (30) is in the first configuration.

3. The instrument of claim 2, wherein the smaller concavity (14) is delimited by a front face (14a) which adheres to the upper zone of the communicating mouth (34) of the compartment, so as to close the majority of the mouth (34).

4. The instrument of claim 1, wherein the plane (F) defined by the communication (34) of the compartment forms an angle (B) with the plane (H) defined by the scraping face (21) of the blade (20), a value of which is around 40 degrees, and preferably exactly 40 degrees.

5. The instrument of claim 1, wherein the axis (M) of the distal end (32) of the compartment forms an angle (D) with the plane (H) defined by the scraping face (21) of the blade (20) a value of which is around 70 degrees, and preferably exactly 70 degrees.

6. The instrument of claim 1, **characterised in that** it comprises a lever (60) hinged to the handle (10), which surrounds the compartment (30) when the compartment (30) is in the first operating configuration,
where the lever (60) has two end positions, in which:
- in the first end position, the lever (60) rests against the compartment (30), in turn positioned restingly against the internal face (13) of the handle, in the first operating configuration,
- in the second end position, the lever (60) is raised from the handle (10) and in turn raises the compartment (30) from the handle (10).

7. The instrument of claim 1, wherein:
- the collecting compartment (30) is solidly joined to a rear support stem (40), a rear end (42) of which is hinged to the proximal end (12) of the handle (10), by means of a hinge having a horizontal rotation axis that is transversal with respect to the horizontal axis (A) of the handle (10);
- the rear support stem (40) has a shorter length with respect to the handle (10) and is conformed such as to be arranged restingly on the handle (10), and in part such as to co-penetrate the internal face of the handle (10), when the compartment (30) is in the first operating configuration;
- the handle has a transversal section substantially over all a length extension thereof, having a concave shape (U-shape), with the concavity thereof facing downwards.

8. The instrument of claim 7, wherein, when in the first operating position, the rear support stem (40) comes to rest against the end (13') of the U-section of the handle, and simultaneously penetrates for a short portion (40') thereof to joint in the delimited cavity of the face (13) of the handle.

## Patentansprüche

1. Chirurgisches Instrument zum Abschaben und Sammeln von Knochenpartikeln, einen länglichen Griff (10), der ein distales Ende aufweist, welches eine Klinge (20) mit einer nach hinten weisenden Schabefläche (21) und einer äußeren Schabekante (22) trägt, die im Verhältnis zum distalen Ende quer nach außen hervorsteht, und ein Sammelfach (30) für abgeschabtes Material umfassend,
wobei das Sammelfach (30) eine Röhrenform aufweist und eine Sammelkammer (31) mit einem konstanten Querschnitt und ein distales Ende (32) umfasst, das eine Mündung (34) aufweist, die mit einer Außenseite in Verbindung steht,
wobei das Sammelfach (30) mit seinem proximalen Ende (33) mit einem proximalen Abschnitt (12) des Griffs (10) verbunden ist, mittels einer Beschränkung, die eine beidseitige Drehung des Griffs (10) und des Fachs (30) ermöglicht und in der Lage ist, zwei verschiedene Betriebsanordnungen einzunehmen:
- eine erste Betriebsanordnung, in der das Sammelfach (30) direkt an einer Innenfläche (13) des Griffs (10) anliegt, die zum Sammelfach (30) weist, wobei dessen distales Ende (32) in der Nähe der Schabekante (22) anhaftend an dem distalen Ende (11) und der Schabefläche (21) der Klinge angewandt ist, so dass das abgeschabte Material am Einlass aufgenommen wird, und
- eine zweite Betriebsanordnung, in der das Fach (30) in einem Abstand zum distalen Ende (11) des Griffs angeordnet ist und dessen Mündung (34) beabstandet zur Klinge (20) und zum Griff (10) angeordnet ist,
und wobei das chirurgische Instrument einen Kolben (50) umfasst, welcher dem Sammelfach (30) zugeordnet ist, wobei der Kolben (50) eine Stange (51) aufweist, die entlang der Sammelkammer (31) des Sammelfachs (30) gleitet, und einen aktivierenden Stößel (52), der am proximalen Ende des Sammelfachs (30) aus der Sammelkammer (31) hervorsteht.

2. Instrument nach Anspruch 1, wobei das distale Ende (11) des Griffs (10) eine hin zum Sammelfach (30) weisende kleinere Wölbung (14) aufweist, die komplementär zu dem oberen Abschnitt des distalen Endes (32) des Fachs und zu dessen Mündung (34) angepasst ist, so dass die Außenfläche des Abschnitts an der kleineren Wölbung (14) anhaften kann, wenn sich das Fach (30) in der ersten Anordnung befindet.

3. Instrument nach Anspruch 2, wobei die kleinere Wölbung (14) durch eine Vorderfläche (14a) begrenzt ist, die an der oberen Zone der Verbindungsmündung (34) des Fachs anhaftet, so dass der Großteil der Mündung (34) verschlossen ist.

4. Instrument nach Anspruch 1, wobei die Ebene (F) die durch die Verbindung (34) des Fachs definiert ist, mit der Ebene (H), die durch die Schabefläche (21) der Klinge (20) definiert ist, einen Winkel (B) bildet, dessen Größe etwa 40 Grad beträgt und vorzugsweise genau 40 Grad.

5. Instrument nach Anspruch 1, wobei die Achse (M) des distalen Endes (32) des Fachs mit der Ebene (H), die durch die Schabefläche (21) der Klinge (20) definiert ist, einen Winkel (D) bildet, dessen Größe etwa 70 Grad beträgt und vorzugsweise genau 70 Grad.

6. Instrument nach Anspruch 1, **dadurch gekennzeichnet, dass** es einen Hebel (60) umfasst, der an den Griff (10) angelenkt ist und das Fach (30) umgibt, wenn sich das Fach (30) in der ersten Betriebsanordnung befindet,
wobei der Hebel (60) zwei Endpositionen aufweist, wobei:
- der Hebel (60) in der ersten Endposition an dem Fach (30) anliegt, das in der ersten Betriebsanordnung wiederum anliegend an der Innenfläche (13) des Griffs positioniert ist,
- der Hebel (60) in der zweiten Endposition von dem Griff (10) abgehoben ist und wiederum das Fach (30) von dem Griff (10) abhebt.

7. Instrument nach Anspruch 1, wobei:
- das Sammelfach (30) fest mit einem hinteren Stützschaft (40) verbunden ist, dessen hinteres Ende (42) an das proximale Ende (12) des Griffs (10) angelenkt ist, mittels eines Gelenks, das eine horizontale Drehachse aufweist, die quer zur horizontalen Achse (A) des Griffs (10) liegt,
- der hintere Stützschaft (40) eine im Verhältnis zum Griff (10) kürzere Länge aufweist und derart angepasst ist, dass er an dem Griff (10) anliegend angeordnet ist und teilweise derart, dass er mit der Innenfläche des Griffs (10) in Eingriff gelangt, wenn sich das Fach (30) in der ersten Betriebsanordnung befindet,
- der Griff im Wesentlichen über seine gesamte Längenerstreckung einen Querschnitt mit einer konkaven Form (U-Form) aufweist, deren Wölbung nach unten weist.

8. Instrument nach Anspruch 7, wobei der hintere Stützschaft (40) in der ersten Betriebsanordnung zum Anliegen an dem Ende (13') des U-Abschnitts des Griffs kommt und gleichzeitig mit einem kurzen Abschnitt (40') von ihm in Eingriff gelangt, um sich mit dem begrenzten Hohlraum der Fläche (13) des Griffs zu verbinden.

## Revendications

1. Instrument chirurgical pour racler et collecter des particules sur des os, comprenant une poignée allongée (10), disposant d'une extrémité distale portant une lame (20) disposant d'une face racleuse (21), orientée vers l'arrière, et un racloir externe (22), faisant saillie dans le sens transversal et vers l'extérieur par rapport à l'extrémité distale, et un compartiment collecteur (30) pour les matières raclées,
dans lequel
le compartiment collecteur (30) possède une forme tubulaire et comprend une chambre collectrice (31) disposant d'une section transversale régulière et d'une extrémité distale (32) qui possède une ouverture (34) communiquant avec l'extérieur,
le compartiment collecteur (30) est relié par une extrémité proximale (33) de ce dernier à une partie proximale (12) de la poignée (10), au moyen d'une contrainte qui permet une rotation réciproque de la poignée (10) et du compartiment (30), et peut être placé dans deux configurations de fonctionnement différentes :
- une première configuration de fonctionnement dans laquelle le compartiment collecteur (30) repose directement sur une face interne (13) de la poignée (10) face au compartiment collecteur (30), avec une extrémité distale (32) de ce dernier adhérant à l'extrémité distale (11) et orientée vers la face racleuse (21) de la lame, à proximité du racloir (22), afin de recevoir les matières raclées dans l'entrée, et
- une seconde configuration de fonctionnement dans laquelle le compartiment (30) est situé à distance de l'extrémité distale (11) de la poignée et une ouverture (34) de ce dernier est située à distance de la lame (20) et de la poignée (10),
et dans lequel l'instrument chirurgical comprend
un piston (50) associé au compartiment collecteur (30), dans lequel le piston (50) possède une tige (51) qui coulisse le long de la chambre collectrice (31) du compartiment collecteur (30) et un piston-plongeur d'activation (52) faisant saillie sur la chambre collectrice, au niveau de l'extrémité proximale du compartiment collecteur (30).

2. Instrument selon la revendication 1, dans lequel l'extrémité distale (11) de la poignée (10) présente une plus petite concavité (14) orientée vers le compartiment collecteur (30), complémentairement conforme à la partie supérieure de l'extrémité distale (32) du compartiment et à l'ouverture (34) de ce dernier, de sorte que la surface externe de la partie puisse adhérer à la plus petite concavité (14), lorsque le compartiment est placé dans la première configuration.

3. Instrument selon la revendication 2, dans lequel la plus petite concavité (14) est délimitée par une face avant (14a) qui adhère à la zone supérieure de l'ouverture (34) communicante du compartiment, afin de fermer la majeure partie de la bouche (34).

4. Instrument selon la revendication 1, dans lequel le plan (F) défini par la communication (34) du compartiment forme un angle (B) avec le plan (H) défini par la face racleuse (21) de la lame (20), d'environ 40 degrés, et de préférence d'exactement 40 degrés.

5. Instrument selon la revendication 1, dans lequel l'axe (M) de l'extrémité distale (32) du compartiment forme un angle (D) avec le plan (H) défini par la face racleuse (21) de la lame (20), d'environ 70 degrés, et de préférence d'exactement 70 degrés.

6. Instrument selon la revendication 1, **caractérisé en ce qu'**il comprend un levier (60) articulé sur la poignée (10), qui encercle le compartiment (30) lorsque le compartiment (30) est placé dans la première configuration de fonctionnement,
où le levier (60) possède deux positions extrêmes, dans lesquelles :
- dans la première position extrême, le levier (60) repose sur le compartiment (30), à son tour positionné en appui sur la face interne (13) de la poignée, dans la première configuration de fonctionnement,
- dans la seconde position extrême, le levier (60) est surélevé par rapport à la poignée (10) et à son tour surélève le compartiment (30) par rapport à la poignée.

7. Instrument selon la revendication 1, dans lequel :
- le compartiment collecteur (30) est solidement raccordé à une tige de support arrière (40), dont une extrémité arrière (42) est articulée sur l'extrémité proximale (12) de la poignée (10), au moyen d'une articulation disposant d'un axe de rotation horizontal qui est transversal par rapport à l'axe horizontal (A) de la poignée (10) ;
- la tige de support arrière (40) est plus courte que la poignée (10) et est conçue de sorte qu'elle puisse reposer sur la poignée (10), et en partie de façon à copénétrer la face interne de la poignée (10), lorsque le compartiment (30) est dans la première configuration de fonctionnement ;
- la poignée dispose d'une partie transversale sur sensiblement toute une extension de longueur de ce dernier, disposant d'une forme concave (en U), avec sa concavité orientée vers le bas.

8. Instrument selon la revendication 7, dans lequel, lorsqu'il est dans la première position de fonctionnement, la tige de support arrière (40) vient reposer sur l'extrémité (13') de la partie en U de la poignée, et pénètre simultanément sur une petite partie (40') de ce dernier pour s'articuler dans la cavité délimitée de la face (13) de la poignée.
